# EUROPEAN PATENT SPECIFICATION

(11) **EP 4 063 498 B1**
(45) Date of publication and mention of the grant of the patent: **24.04.2024**
(21) Application number: 21870557.2
(22) Date of filing: 03.12.2021
(51) Int. Cl.: C07K 16/12, C07K 16/40, C12N 5/20, G01N 33/573

(54) **ANTI-OXA-23 TYPE CARBAPENASE HYBRIDOMA CELL LINE, MONOCLONAL ANTIBODY, AND APPLICATION**
OXA-23-TYP CARBAPENEMASE-RESISTENTE HYBRIDOMA-ZELLLINIE UND VERWENDUNG VON MONOKLONALEN ANTIKÖRPERN (MAB) DAVON
LIGNÉE CELLULAIRE D'HYBRIDOME DE CARBAPÉNÉMASE DE TYPE ANTI-OXA-23, ANTICORPS MONOCLONAL ET APPLICATION

(30) Priority: 07.02.2021 CN 202110166073
(43) Date of publication of application: 28.09.2022
(73) Proprietor: Tianjin Era Biology Technology Co., Ltd., Tianjin 300457 (CN); BEIJING GOLD MOUNTAINRIVER TECH DEVELOPMENT CO., LTD., Beijing 100081 (CN)
(72) Inventor: LI, Keke, Tianjin 300480 (CN); HE, Yongsheng, Tianjin 300480 (CN); YUAN, Qinghua, Tianjin 300480 (CN); CHEN, Xiaoling, Tianjin 300480 (CN); ZHOU, Yuehui, Tianjin 300480 (CN); FAN, Linlin, Tianjin 300480 (CN); WANG, Xing, Tianjin 300480 (CN); ZANG, Danrong, Tianjin 300480 (CN)
(74) Representative: Bayramoglu et al.
(86) International application number: PCT/CN2021/135404
(87) International publication number: WO 2022/166354

(56) References cited:
- CN-A- 107 667 291
- CN-A- 112 500 489
- CN-A- 112 501 131
- CN-A- 112 980 803
- CN-A- 112 980 804
- US-A1- 2014 356 859
- MERTINS SONJA ET AL: "Generation and selection of antibodies for a novel immunochromatographic lateral flow test to rapidly identify OXA-23-like-mediated carbapenem resistance in Acinetobacter baumannii", JOURNAL OF MEDICAL MICROBIOLOGY, vol. 68, no. 7, 1 July 2019 (2019-07-01), pages 1021-1032, XP093007047, ISSN: 0022-2615, DOI: 10.1099/jmm.0.001015 Retrieved from the Internet: URL:https://www.microbiologyresearch.org/d ocserver/fulltext/jmm/68/7/1021_jmm001015. pdf?expires=1670841978&id=id&accname=guest &checksum=A2AE8AF9096340C75468B982C23B23BE >
- Zhang Bao-Rong, Bi Ru-Ru;Gu Bing: "Advances in phenotypic detection methods for carbapenemase production in Enterobacteriaceae", Chinese Journal of Infection Control, vol. 17, no. 2, 1 February 2018 (2018-02-01), pages 175-180, XP055955535, ISSN: 1671-9638, DOI: 10.3969/j.issn.1671-9638.2018.02.019
- Schuertz Kamile Francine, Tuon Felipe Francisco, Palmeiro Jussara Kasuko, Conte Danieli, Telles João Paulo Marochi, Trevisoli Luca: "Bacteremia and meningitis caused by OXA-23-producing Acinetobacter baumannii ? molecular characterization and susceptibility testing for alternative antibiotics", BRAZILIAN JOURNAL OF MICROBIOLOGY, Sociedade Brasileira de Microbiologia,Brazilian Society for Microbiology, BR, vol. 49, no. Suppl. 1, 1 November 2018 (2018-11-01), pages 199-204, XP055955536, BR ISSN: 1517-8382, DOI: 10.1016/j.bjm.2018.04.002

## Description

### TECHNICAL FIELD

The present disclosure belongs to the technical field of antibody preparation, and in particular to an OXA-23-type carbapenemase-resistant hybridoma cell line, and a monoclonal antibody (mAb) and use thereof.

### BACKGROUND

Among β-lactam antibiotics, carbapenems have the most extensive antibacterial activities, can resist the hydrolysis of most β-lactamases and extended spectrum beta-lactamases (ESBLs), and can attenuate the chromosomally-mediated action of Amp C enzymes. *Acinetobacter* shows some resistance to many antibiotics such as β-lactams, aminoglycosides, and fluoroquinolones, while carbapenems have always shown high antibacterial activity to *Acinetobacter.* However, with the widespread application of carbapenems in clinical practice, a resistance rate of *Acinetobacter baumannii* (*A. baumannii*) to carbapenems is increasing year by year. In recent years, there have been continuous reports on nosocomial infection caused by *Acinetobacter* carrying blaOXA-23. It has been reported in the literature that 80% of carbapenem-resistant A. *baumannii* in China produces OXA-23-type carbapenemase, which makes the clinical treatment of carbapenem-resistant *Acinetobacter* very difficult. According to 2011 data of the China Antimicrobial Surveillance Network (CHINET) of the Ministry of Health, resistance rates of *A. baumannii* to carbapenems imipenem and meropenem in China were 56.8% and 58.7%, respectively, indicating that carbapenem-resistant *A. baumannii* poses a serious challenge to clinical anti-infective treatment.

There are numerous methods for laboratory detection of carbapenemases. The methods mainly include modified Hodge test, Carba NP test, modified carbapenem inactivation method (mCIM), enzyme inhibitor enhancement test, immunogold labeling test, molecular biotechnology, and the like. Immunodiagnostic reagents are the fastest-growing segment of *in vitro* diagnostic reagents, which achieve qualitative or quantitative detection through specific binding between an antigen and an antibody.

Mertins Sonja et. al, discloses an immunochromatographic lateral flow test (ICT) for the rapid and reliable detection of OXA-23-producing carbapenem-resistant Acinetobacter isolates (MERTINS SONJA ET AL: "Generation and selection of antibodies for a novel immunochromatographic lateral flow test to rapidly identify OXA-23-like-mediated carbapenem resistance in Acinetobacter baumannii", JOURNAL OF MEDICAL MICROBIOLOGY, vol. 68, no. 7, 1 July 2019, pages 1021-1032). However, it does not disclose the sensitivity of the antibody.

### SUMMARY

The present disclosure provides an OXA-23-type carbapenemase-resistant hybridoma cell line, and a mAb and use thereof.

The present disclosure adopts the following technical solutions:
An anti-OXA-23-type carbapenemase antibody is provided, wherein the anti-OXA-23-type carbapenemase antibody is: an antibody HB12 including a light chain variable region and a heavy chain variable region, where complementary-determining regions (CDRs) of the light chain variable region include three sequences shown in SEQ ID NO: 1, SEQ ID NO: 2, and SEQ ID NO: 3; and CDRs of the heavy chain variable region include three sequences shown in SEQ ID NO: 4, SEQ ID NO: 5, and SEQ ID NO: 6;
SEQ ID NO: 1
   KASQDVSIAVA (CDR-L1)
SEQ ID NO: 2
   WASTRHT (CDR-L2)
SEQ ID NO: 3
   QQHYSTPYL (CDR-L3)
SEQ ID NO: 4
   GYTFTSYWMY (CDR-H1)
SEQ ID NO: 5
   EIYPTNGRTNYNEKFKT (CDR-H2)
SEQ ID NO: 6
   YYYGSYAMDY (CDR-H3)
   or,
an antibody FA2 including a light chain variable region and a heavy chain variable region, where CDRs of the light chain variable region include three sequences shown in SEQ ID NO: 11, SEQ ID NO: 12, and SEQ ID NO: 13; and CDRs of the heavy chain variable region include three sequences shown in SEQ ID NO: 14, SEQ ID NO: 15, and SEQ ID NO: 16.
SEQ ID NO: 11
   KGSQDVRTAVA (CDR-L1)
SEQ ID NO: 12
   WASTRHT (CDR-L2)
SEQ ID NO: 13
   QQHYSIPYT (CDR-L3)
SEQ ID NO: 14
   GFSLTSYGVH (CDR-H1)
SEQ ID NO: 15
   VIWRGGNTDYNAAFMSRLS (CDR-H2)
SEQ ID NO: 16
   SLTTSTFDY (CDR-H3)
In the antibody HB 12, the light chain variable region may have an amino acid sequence shown in SEQ ID NO: 7, and the heavy chain variable region may have an amino acid sequence shown in SEQ ID NO: 9;
SEQ ID NO: 7 amino acid sequence of light chain variable region 
DIVMTQSHKFMSTSVGDRVSITCKASQDVSIAVAWFQQKPGQSPKLLIYWASTR
HTGVPDRFTGSGSGTDYTLTISSVQAEDLALYYCQQHYSTPYLFGGGTKLEIKRA
SEQ ID NO: 9 amino acid sequence of heavy chain variable region 
VQLQQSGAELVKPGASVKLSCKTSGYTFTSYWMYWVKQRPGQGLEWIGEIYPT
NGRTNYNEKFKTKATLTVDKSSSTAYMQLSSLTSEDSAVYYCAIYYYGSYAMDYWG
QGTSVTVSS
 or,
in the antibody FA2, the light chain variable region may have an amino acid sequence shown in SEQ ID NO: 17, and the heavy chain variable region may have an amino acid sequence shown in SEQ ID NO: 19.
SEQ ID NO: 17 amino acid sequence of light chain variable region 
DIVMTQSHKLMSTSVGDRVSITCKGSQDVRTAVAWYQQKPGQSPNLLIYWASTR
HTGVPDRFTGSGSGTDYILTITSVQAEDLALYYCQQHYSIPYTFGGGTKLEIKRA
SEQ ID NO: 19 amino acid sequence of heavy chain variable region

VQLKQSGPGLVQPSQSLSITCTVSGFSLTSYGVHWVRRSPGKGLEWLGVIWRGG
NTDYNAAFMSRLSITKDNSKSQVFFKMTSLQGDDTAIYSCVKSLTTSTFDYWGQGTL
VTVSS

A nucleic acid is provided, including a nucleotide sequence encoding the anti-OXA-23-type carbapenemase antibody described above.

Preferably, a nucleotide sequence of the nucleic acid for encoding the light chain variable region of the antibody HB 12 may be as shown in SEQ ID NO: 8, and a nucleotide sequence of the nucleic acid for encoding the heavy chain variable region of the antibody HB12 may be as shown in SEQ ID NO: 10; and
SEQ ID NO: 8 base sequence for light chain variable region 
GACATTGTGATGACCCAGTCTCACAAATTCATGTCCACATCAGTAGGAGACA
GGGTCAGCATCACCTGCAAGGCCAGTCAGGATGTGAGTATTGCTGTAGCCTGGTT
TCAACAAAAACCAGGGCAATCTCCTAAACTACTGATTTACTGGGCATCCACCCGG
CACACTGGAGTCCCTGATCGCTTCACAGGCAGTGGATCTGGGACAGATTATACTC
TCACCATCAGCAGTGTGCAGGCTGAAGACCTGGCACTTTATTACTGTCAGCAACA
TTATAGCACTCCGTATTTGTTCGGAGGGGGGACCAAGCTGGAAATAAAACGGGCT
SEQ ID NO: 10 base sequence for heavy chain variable region 
GTGCAGCTTCAGCAGTCAGGGGCTGAACTGGTGAAGCCTGGGGCCTCAGTGA
AGCTGTCCTGCAAGACTTCTGGCTACACCTTCACCAGCTACTGGATGTACTGGGT
GAAGCAGAGGCCTGGACAAGGCCTTGAGTGGATTGGAGAGATTTATCCTACCAA
CGGTCGTACTAACTACAATGAGAAATTCAAGACCAAGGCCACACTGACTGTGGA
CAAATCCTCCAGCACAGCCTACATGCAACTCAGCAGCCTGACATCTGAGGACTCT
GCGGTCTATTACTGTGCAATTTATTACTACGGTAGCTATGCTATGGACTACTGGGG
TCAAGGAACCTCAGTCACCGTCTCCTCA
 or,
a nucleotide sequence of the nucleic acid for encoding the light chain variable region of the antibody FA2 may be as shown in SEQ ID NO: 18, and a nucleotide sequence of the nucleic acid for encoding the heavy chain variable region of the antibody FA2 may be as shown in SEQ ID NO: 20.
SEQ ID NO: 18 base sequence for light chain variable region 
GACATTGTGATGACCCAGTCTCACAAACTCATGTCCACATCAGTAGGAGACA
GGGTCAGCATCACCTGCAAGGCCAGTCAGGATGTGAGGACTGCTGTAGCCTGGT
ATCAACAAAAACCAGGACAATCTCCTAACCTACTGATTTACTGGGCATCCACCCG
GCACACTGGAGTCCCTGATCGCTTCACAGGCAGTGGATCTGGGACAGATTACATT
CTCACCATCACTAGTGTGCAGGCTGAAGACCTGGCACTTTATTACTGTCAGCAAC
ATTATAGTATTCCTTACACGTTCGGAGGGGGGACCAAGCTGGAAATAAAACGGG
CT
SEQ ID NO: 20 base sequence for heavy chain variable region

GTGCAACTGAAGCAGTCAGGACCTGGCCTAGTGCAGCCCTCACAGAGTCTGT
CCATAACCTGCACAGTCTCTGGTTTCTCATTAACTAGCTATGGTGTACACTGGGTT
CGCCGGTCTCCAGGAAAGGGTCTGGAGTGGCTGGGAGTGATCTGGAGAGGTGGA
AACACAGACTACAATGCAGCTTTCATGTCCAGACTGAGCATCACCAAGGACAACT
CCAAGAGCCAAGTTTTCTTTAAAATGACCAGTCTGCAAGGTGATGACACCGCCAT
ATACTCCTGTGTCAAATCCCTTACTACGTCTACGTTTGATTACTGGGGCCAAGGGA
CTCTGGTCACCGTCTCCTCA

Use of the anti-OXA-23-type carbapenemase antibody described above in the detection of an OXA-23-type carbapenemase antigen is provided.

Preferably, the anti-OXA-23-type carbapenemase antibody may be used in an *in vitro* diagnostic kit or a microfluidic chip; and the *in vitro* diagnostic kit may be a colloidal gold immunoassay kit, a chemiluminescence kit, a radioimmunoassay kit, an enzyme-linked immunosorbent assay (ELISA) kit, or a fluoroimmunoassay kit.

Preferably, an immune colloidal gold test strip of a double-antibody sandwich method may be prepared, wherein the antibody HB 12 is used as a coating antibody and the antibody FA2 is used as a gold-labeled antibody; or the antibody HB 12 is used as a gold-labeled antibody and the antibody FA2 is used as a coating antibody.

The present disclosure has the following advantages and positive effects: An anti-OXA-23-type carbapenemase antibody is provided, which can be used to detect OXA-23-type carbapenemase, with a titer of more than 1 :640,000; an *in vitro* diagnostic kit or a microfluidic chip prepared therefrom can be used for early typing of drug-resistant strains, guidance of drug use, and assistance of clinical infection control and treatment.

### BRIEF DESCRIPTION OF THE DRAWINGS

FIG. 1 is an electrophoretogram of the OXA-23-type carbapenemase protein; and
FIG. 2 is an electrophoretogram of the anti-OXA-23-type carbapenemase antibody protein.

### DETAILED DESCRIPTION OF THE EMBODIMENTS

The embodiments of the present disclosure will be described below.

The present disclosure relates to an OXA-23-type carbapenemase-resistant hybridoma cell line named HB12. The present disclosure also relates to an OXA-23-type carbapenemase-resistant hybridoma cell line named FA2.

The OXA-23-type carbapenemase-resistant hybridoma cell line capable of stably secreting an anti-OXA-23-type carbapenemase antibody and a variable region sequence thereof provided by the present disclosure are obtained through mouse hybridoma mAb screening and reverse transcription-polymerase chain reaction (RT-PCR) to clone an Ig variable region gene, and the binding specificity of the antibody is identified by ELISA.

An antibody HB 12 produced by the OXA-23-type carbapenemase-resistant hybridoma cell line includes a light chain variable region and a heavy chain variable region, where CDRs of the light chain variable region include three sequences shown in SEQ ID NO: 1, SEQ ID NO: 2, and SEQ ID NO: 3;
SEQ ID NO: 1
   KASQDVSIAVA (CDR-L1)
SEQ ID NO: 2
   WASTRHT (CDR-L2)
SEQ ID NO: 3
   QQHYSTPYL (CDR-L3)
CDRs of the heavy chain variable region include three sequences shown in SEQ ID NO: 4, SEQ ID NO: 5, and SEQ ID NO: 6;
SEQ ID NO: 4
   GYTFTSYWMY (CDR-H1)
SEQ ID NO: 5
   EIYPTNGRTNYNEKFKT (CDR-H2)
SEQ ID NO: 6
   YYYGSYAMDY (CDR-H3)
the light chain variable region may have an amino acid sequence shown in SEQ ID NO: 7;
SEQ ID NO: 7 amino acid sequence of light chain variable region 
DIVMTQSHKFMSTSVGDRVSITCKASQDVSIAVAWFQQKPGQSPKLLIYWASTR
HTGVPDRFTGSGSGTDYTLTISSVQAEDLALYYCQQHYSTPYLFGGGTKLEIKRA
the heavy chain variable region may have an amino acid sequence shown in SEQ ID NO: 7;
SEQ ID NO: 9 amino acid sequence of heavy chain variable region 
VQLQQSGAELVKPGASVKLSCKTSGYTFTSYWMYWVKQRPGQGLEWIGEIYPT
NGRTNYNEKFKTKATLTVDKSSSTAYMQLSSLTSEDSAVYYCAIYYYGSYAMDYWG
QGTSVTVSS
a nucleotide sequence encoding the light chain variable region of the anti-OXA-23-type carbapenemase antibody may be as shown in SEQ ID NO: 8; and
SEQ ID NO: 8 
GACATTGTGATGACCCAGTCTCACAAATTCATGTCCACATCAGTAGGAGACA
GGGTCAGCATCACCTGCAAGGCCAGTCAGGATGTGAGTATTGCTGTAGCCTGGTT
TCAACAAAAACCAGGGCAATCTCCTAAACTACTGATTTACTGGGCATCCACCCGG
CACACTGGAGTCCCTGATCGCTTCACAGGCAGTGGATCTGGGACAGATTATACTC
TCACCATCAGCAGTGTGCAGGCTGAAGACCTGGCACTTTATTACTGTCAGCAACA
TTATAGCACTCCGTATTTGTTCGGAGGGGGGACCAAGCTGGAAATAAAACGGGCT
a nucleotide sequence encoding the heavy chain variable region of the anti-OXA-23-type carbapenemase antibody may be as shown in SEQ ID NO: 10.
SEQ ID NO: 10

GTGCAGCTTCAGCAGTCAGGGGCTGAACTGGTGAAGCCTGGGGCCTCAGTGA
AGCTGTCCTGCAAGACTTCTGGCTACACCTTCACCAGCTACTGGATGTACTGGGT
GAAGCAGAGGCCTGGACAAGGCCTTGAGTGGATTGGAGAGATTTATCCTACCAA
CGGTCGTACTAACTACAATGAGAAATTCAAGACCAAGGCCACACTGACTGTGGA
CAAATCCTCCAGCACAGCCTACATGCAACTCAGCAGCCTGACATCTGAGGACTCT
GCGGTCTATTACTGTGCAATTTATTACTACGGTAGCTATGCTATGGACTACTGGGG
TCAAGGAACCTCAGTCACCGTCTCCTCA

An antibody FA2 produced by the OXA-23-type carbapenemase-resistant hybridoma cell line includes a light chain variable region and a heavy chain variable region, where CDRs of the light chain variable region include three sequences shown in SEQ ID NO: 11, SEQ ID NO: 12, and SEQ ID NO: 13;
SEQ ID NO: 11
   KGSQDVRTAVA (CDR-L1)
SEQ ID NO: 12
   WASTRHT (CDR-L2)
SEQ ID NO: 13
   QQHYSIPYT (CDR-L3)
CDRs of the heavy chain variable region include three sequences shown in SEQ ID NO: 14, SEQ ID NO: 15, and SEQ ID NO: 16;
SEQ ID NO: 14
   GFSLTSYGVH (CDR-H1)
SEQ ID NO: 15
   VIWRGGNTDYNAAFMSRLS (CDR-H2)
SEQ ID NO: 16
   SLTTSTFDY (CDR-H3)
the light chain variable region may have an amino acid sequence shown in SEQ ID NO: 17;
SEQ ID NO: 17 amino acid sequence of light chain variable region 
DIVMTQSHKLMSTSVGDRVSITCKGSQDVRTAVAWYQQKPGQSPNLLIYWASTR
HTGVPDRFTGSGSGTDYILTITSVQAEDLALYYCQQHYSIPYTFGGGTKLEIKRA
the heavy chain variable region may have an amino acid sequence shown in SEQ ID NO: 19;
SEQ ID NO: 19 amino acid sequence of heavy chain variable region 
VQLKQSGPGLVQPSQSLSITCTVSGFSLTSYGVHWVRRSPGKGLEWLGVIWRGG
NTDYNAAFMSRLSITKDNSKSQVFFKMTSLQGDDTAIYSCVKSLTTSTFDYWGQGTL
VTVSS
a nucleotide sequence encoding the light chain variable region of the anti-OXA-23-type carbapenemase antibody may be shown in SEQ ID NO: 18; and
SEQ ID NO: 18 
GACATTGTGATGACCCAGTCTCACAAACTCATGTCCACATCAGTAGGAGACA
GGGTCAGCATCACCTGCAAGGGCAGTCAGGATGTGAGGACTGCTGTAGCCTGGT
ATCAACAAAAACCAGGACAATCTCCTAACCTACTGATTTACTGGGCATCCACCCG
GCACACTGGAGTCCCTGATCGCTTCACAGGCAGTGGATCTGGGACAGATTACATT
CTCACCATCACTAGTGTGCAGGCTGAAGACCTGGCACTTTATTACTGTCAGCAAC
ATTATAGTATTCCTTACACGTTCGGAGGGGGGACCAAGCTGGAAATAAAACGGG
CT
a nucleotide sequence encoding the heavy chain variable region of the anti-OXA-23-type carbapenemase antibody may be shown in SEQ ID NO: 20.
SEQ ID NO: 20

GTGCAACTGAAGCAGTCAGGACCTGGCCTAGTGCAGCCCTCACAGAGTCTGT
CCATAACCTGCACAGTCTCTGGTTTCTCATTAACTAGCTATGGTGTACACTGGGTT
CGCCGGTCTCCAGGAAAGGGTCTGGAGTGGCTGGGAGTGATCTGGAGAGGTGGA
AACACAGACTACAATGCAGCTTTCATGTCCAGACTGAGCATCACCAAGGACAACT

CCAAGAGCCAAGTTTTCTTTAAAATGACCAGTCTGCAAGGTGATGACACCGCCAT
ATACTCCTGTGTCAAATCCCTTACTACGTCTACGTTTGATTACTGGGGCCAAGGGA
CTCTGGTCACCGTCTCCTCA

The anti-OXA-23-type carbapenemase antibodies produced by the hybridoma cell line HB12 and the hybridoma cell line FA2 can be used to detect an OXA-23-type carbapenemase antigen. The anti-OXA-23-type carbapenemase mAb shows prominent performance in all aspects, and thus is suitable as an immunodiagnostic reagent for the preparation of an *in vitro* diagnostic kit, and the *in vitro* diagnostic kit may be a colloidal gold immunoassay kit, a chemiluminescence kit, a radioimmunoassay kit, an ELISA kit, or a fluoroimmunoassay kit. Alternatively, the anti-OXA-23-type carbapenemase antibody can be made into a microfluidic chip for detecting an OXA-23-type carbapenemase antigen.

To develop an OXA-23-type carbapenemase diagnostic reagent of the colloidal gold method, an OXA-23-type carbapenemase antigen first needs to be extracted to obtain a high-purity antigen, then the antigen is used to stimulate an excellent immune response in mice, and then the hybridoma technique is used to screen an antibody with high affinity and specificity for the development of related *in vitro* diagnostic reagents. The two antibodies involved in this solution are especially suitable for preparing an immune colloidal gold test strip of a double-antibody sandwich method, wherein the antibody 1AA4 is used as a coating antibody and the antibody AH9 is used as a gold-labeled antibody. The prepared immune colloidal gold test strip of a double-antibody sandwich method is more sensitive. Moreover, the antibody HB12 can be used as a gold-labeled antibody, and the antibody FA2 can be used as a coating antibody. The present disclosure is further described below through specific examples. Experimental methods for which operation steps are not specified are all conducted in accordance with the corresponding product instructions. The instruments, reagents, and consumables used in the examples can be purchased from commercial companies unless otherwise specified.

### Example 1: Preparation of an anti-OXA-23-type carbapenemase antibody

### 1.1 Antigen expression

Sequence acquisition: An OXA-23-type gene sequence was subjected to gene synthesis and ligated with a vector Pet-28a (+).

Transformation into host bacteria: The vector Pet-28a (+) carrying the target gene was transformed into a cloning host *Escherichia coli* (*E. coli*) (Rosetta^{™} (DE3) bacteria of BL21 series).

Expansion cultivation: Verified positive transformants were subjected to expansion cultivation, then transferred to a kanamycin-resistant LB liquid medium at a ratio of 1:100, and cultivated at 37°C under shaking on a shaker.

Induction: When an OD value of a bacterial solution was of 0.4 to 0.6, isopropylthio-β-galactoside (IPTG) was added at a final concentration of 1 mM, then the bacteria were further cultivated at 37°C for 3 h, and bacterial cells were collected by centrifugation.

Ultrasonication of bacterial cells: The bacterial cells were resuspended by adding 30 mL of phosphate buffered saline (PBS) to every about 1 g of the bacterial cells, and then subjected to ultrasonication at a power of 400 W for about 30 min (ultrasonic time: 3 s, interval: 5 s); after a bacterial solution was non-viscous and clear, the bacterial solution was centrifuged to remove bacterial debris, and a resulting supernatant was filtered through a 0.45 µm filter membrane.

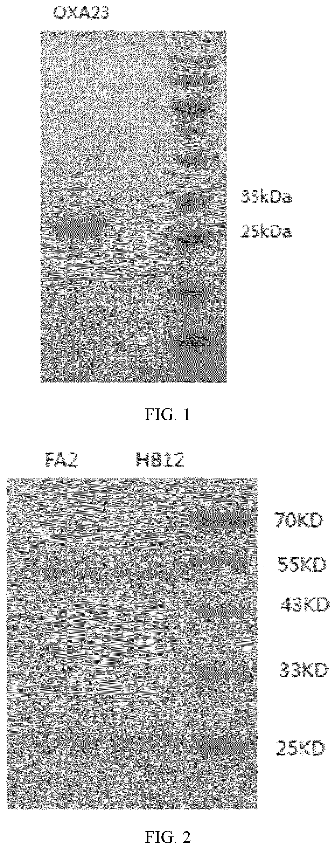

Purification: The supernatant was loaded on a nickel column packed and equilibrated with an equilibration buffer in advance. After the loading was completed, gradient elution was conducted with imidazole eluents and washing liquids at concentrations of 150 mM and 250 mM. When the imidazole concentration was 500 mM, a target protein with high purity could be obtained, and a molecular weight thereof was consistent with the expected 33 kD. A sodium dodecyl sulphate-polyacrylamide gel electrophoresis (SDS-PAGE) result was shown in FIG. 1. The protein was quantified by bicinchoninic acid (BCA) assay and then dispensed for subsequent experiments.

### 1.2 Mouse immunization

6-week-old female Balb/c mice were immunized with the purified OXA-23-type carbapenemase to prepare an antibody, with a protein content of 0.1 mg/ml. The mice were divided into 2 groups according to the immunization dosage, with 5 mice in each group; according to a protein content, a first group was immunized at an immunization dosage of 25 µg/mouse, and a second group was immunized at an immunization dosage of 50 µg/mouse. For an initial immunization, an appropriate amount of the OXA-23-type carbapenemase was taken and diluted with distilled water to 300 µL, then 300 µL of complete Freund's adjuvant (CFA) was added for thorough emulsification, and a resulting emulsion was subcutaneously injected into the mice (multi-point injection); two weeks later, the mice were subjected to a secondary immunization by intraperitoneal injection at the same dosage; two weeks later, the mice were subjected to an additional immunization by intraperitoneal injection; 7 days later, blood was collected from the tail of mice, and a serum titer of the mice was determined by ELISA.

Specific steps were as follows: An ELISA plate was coated overnight at 4°C with the OXA-23-type carbapenemase of 0.2 µg/ml at 100 µL/well, spin-dried, and washed 3 times with phosphate-buffered saline with Tween 20 (PBST). 5% skimmed milk powder was added at 200 µL/well to block at 37°C for 2 h. Blood was collected from the tail of the mice and then centrifuged at 3,000 rpm to obtain serum, and the serum was serially diluted from 1: 1,000 to 1:512,000 with PBS for later use. The plate was spin-dried and then washed 3 times with PBST, a primary antibody diluted with PBS at 1:1,000 was added at 100 µL/well, and the plate was incubated at 37°C for 1 h. The plate was spin-dried and then washed 3 times with PBST, a goat anti-mouse secondary antibody diluted with PBS at 1:6,000 was added at 100 µL/well, and the plate was incubated at 37°C for 45 min. The plate was spin-dried and then washed 5 times with PBST, 3,3',5,5'-tetramethylbenzidine (TMB) was added at 100 µL/well, and the plate was incubated at 37°C to allow a chromogenic reaction for 10 min; the chromogenic reaction was stopped, and a value was read.

### 1.3 Cell fusion

Three days before fusion, mice were subjected to a booster immunization by intraperitoneal injection at the same dosage as the previous immunization, without an adjuvant. Feeder cells were prepared one day before fusion. A Balb/c mouse 6-8 weeks old was selected, and eyeballs of the mouse were removed to collect blood; then the mouse was sacrificed by cervical dislocation, disinfected in 75% alcohol for 5 min, and fixed on a tray; the abdominal skin was aseptically cut in a clean bench. 10 ml of an HAT selection medium was injected into the abdominal cavity of the mouse with a sterile syringe, the abdomen was slightly rubbed with an alcohol cotton ball, and the medium was drawn out. The medium was added to 40 ml of an HAT medium, and a resulting mixture was added to 4 96-well cell culture plates at 100 µL/well, and then cultivated in a 37°C and 5% CO₂ incubator. One week before fusion, myeloma cells (Sp2/0 cells) were recovered, cultivated in a PRMI-1640 medium with 10% fetal bovine serum (FBS), and subcultivated in a 37°C and 5% CO₂ incubator. Cells in a logarithmic growth phase were collected into a centrifuge tube, subjected to cell counting, and diluted to 10⁷ cells/ml for later use. The Balb/c mice that had been subjected to the booster immunization 3 days ago were taken, eyeballs were removed to collect blood and prepare positive serum, and the mice were sacrificed by cervical dislocation and disinfected with 75% alcohol for 5 min; then a spleen was aseptically collected in a clean bench and washed several times in a sterile dish, and a connective tissue was stripped. The spleen was placed on a microporous copper mesh, a fresh RPMI-1640 medium was added, and the medium was first drawn with a syringe and injected from an end of the spleen to blow off spleen cells; after the process was repeated several times, an inner plug of the syringe was used to gently grind the remaining spleen until there was no obvious red tissue mass. A spleen cell suspension in a dish was gently pipetted up and down, then transferred to a 50 ml centrifuge tube, and centrifuged at 1,000 r/min for 5 min to collect spleen cells, and the spleen cells were counted for later use. The immunized mouse spleen cells and Sp2/0 cells were mixed at a cell number ratio of 10:1, added to a 50 ml centrifuge tube, and centrifuged at 1,000 r/min for 5 min, a resulting supernatant was discarded, and the two cells were thoroughly mixed by rubbing gently in the palm of hands. The centrifuge tube was placed in a 100 mL blue-cap bottle filled with 37°C hot water, and then 1 ml of preheated dimethyl sulfoxide (DMSO)/polyethylene glycol (PEG) was added dropwise to the fusion tube within 1 min, during which the DMSO/PEG was added slowly first and then quickly, and the centrifuge tube was gently swirled. Then an antibiotic-free and serum-free RPMI-1640 medium was immediately added to stop the reaction, during which 1 ml of the medium was added in the first minute, 2 ml of the medium was added in the second minute, 3 ml of the medium was added in the third minute, and 4 ml of the medium was added in the fourth minute. A resulting mixture was incubated in a 37°C water bath for 5 min and then centrifuged at 800 r/min for 5 min, and a resulting supernatant was discarded; a resulting precipitate was suspended with HAT, then thoroughly mixed with 40 ml of an HAT selection medium including 20% calf serum preheated at 37°C, and added to a 96-well cell plate with feeder cells at 100 µL/well, and the cell plate was incubated in a 37°C and 5% CO₂ incubator. 7 days later, half of the medium in the cell plate was replaced with fresh HAT medium, and 10 days later, the medium was completely replaced with HT medium. Positive cells in the 96-well plate were subcloned by limiting dilution analysis (LDA): feeder cells were prepared first according to the above method, and hybridoma cells to be cloned were taken and counted, then diluted to 5 to 8 cells/ml with HT medium, added at 100 µL/well to a 96-well cell plate with the feeder cells (the cloning of each hybridoma cell line required a 96-well cell plate), and cultivated in a 37°C and 5% CO₂ incubator; the number of clones in each cell well was counted and marked about 5 days later, the medium was replaced with fresh medium on day 7, and a test was conducted when cells covered 1/3 to 1/2 of the entire bottom surface of each well; after 2 to 3 times of cloning, all cell wells of the 96-well plate were positive, at which point the cells could be expanded, verified, and cryopreserved. Hybridoma cells verified as positive were expanded and cryopreserved. A specific process was as follows: vigorously-growing hybridoma cells in prominent conditions were gently blown off from a flask with antibiotic-free and serum-free DMEM and then centrifuged at 1,000 r/min for 5 min, and a resulting supernatant was discarded; a cryopreservation solution (with 40% RPMI-1640 medium, 50% FBS, and 10% DMSO) was added to make cells uniformly dispersed, and a resulting cell suspension was then dispensed into cell cryopreservation tubes; then the cryopreservation tubes were placed in a cryopreservation box, the cryopreservation box was placed in a -70°C freezer, and one day later, the cryopreservation tubes were transferred to liquid nitrogen and related records were made.

### 1.4 Ascitic fluid preparation

10 to 12-week-old female Balb/c mice were injected intraperitoneally with sterile liquid paraffin at 0.5 mL/mouse, and 7 days later, the mice were injected intraperitoneally with hybridoma cells in a logarithmic growth phase at 5 × 10⁶ cells/mouse. The mice were observed every day. About 7 to 10 days later, abdomens of the mice obviously bulged, at which point the lower abdominal skin was disinfected with a 75% alcohol cotton ball and then the abdominal cavity was punctured with a 16-gauge needle to collect an ascitic fluid. After an ascitic fluid was regenerated and accumulated, the ascitic fluid was collected once again. The collected ascitic fluid was centrifuged at 3,000 r/min for 10 min, and a resulting clear middle layer was collected, filtered with filter paper, dispensed, and stored at -70°C.

### 1.5 Antibody purification

The ascitic fluid was purified with a Protein-G column. Specific steps were as follows: 2 ml (n) of the ascitic fluid was taken and centrifuged at 10,000 g, and a resulting clear layer was collected and thoroughly mixed with 2 ml of a washing buffer (1:1) to obtain a sample; 20% ethanol was passed through the column, then the column was equilibrated with 8 mL of a washing liquid, and the sample was passed through the column at a flow rate of 8 S/drop; the sample was repeatedly loaded 3 times, and then the column was washed with 15 mL of a washing buffer at a flow rate of 8 S/drop; after the washing was completed, elution was conducted with 10 mL of an elution buffer, and after the elution was completed, a pH was adjusted to 7.4 with 1 M Tris PH = 9; then concentration was conducted with a concentration column, and a resulting concentrate was subjected to dialysis overnight at 4°C with PBS in a 50 kD dialysis bag to obtain an antibody HB12 and an antibody FA2.

### Example 2: Identification of the anti-OXA-23-type carbapenemase antibody

### 2.1 Antibody subtype identification

According to the instructions of the SIGMA kit, the mAb subtype identification was conducted by capture-ELISA. Specific steps were as follows: an mAb subtype identification reagent was diluted at 1:1,000, added to an ELISA plate at 100 µL/well, and incubated at 37°C for 1 h; the plate was washed three times with PBST and pat-dried; the antibody was diluted at 1: 1,000 and added at 100 µL/well, and then the plate was incubated at 37°C for 1 h; the plate was washed three times with PBST and pat-dried; an HRP enzyme-labeled goat anti-mouse IgG secondary antibody was diluted at 1:6,000 and added at 100 µL/well, and then the plate was incubated at room temperature for 30 min; a chromogenic reaction was conducted for 10 min to 20 min. A subtype reagent added in a well with an OD450 reading significantly higher than that of other wells indicated the subtype of the mAb. The antibody subtype of the antibody HB12 was IgG2a, and the antibody subtype of the antibody FA2 was IgG2b.

### 2.2 Antibody titer determination

The antibody titer was determined after purification was conducted by indirect ELISA. Specific steps were as follows: OXA-23-type carbapenemase was diluted to 0.2 µg/mL and added to an ELISA plate at 100 µL/well to coat overnight at 4°C, where an uncoated control was set; the plate was spin-dried and then washed 3 times with PBST; 5% skimmed milk powder was added at 200 µL/well to block at 37°C for 2 h; the plate was spin-dried and then washed 3 times with PBST; the antibody (with a concentration of 1 mg/ml) was serially diluted from 1: 1,000 to obtain a total of 12 gradient concentrations, resulting antibody dilutions were each added to the plate at 100 µL/well, and the plate was incubated at 37°C for 1 h, where an uncoated control was set; the plate was spin-dried and then washed 3 times with PBST, a goat anti-mouse secondary antibody diluted with PBS at 1:6,000 was added at 100 µL/well, and the plate was incubated at 37°C for 45 min; the plate was spin-dried and then washed 5 times with PBST, TMB was added at 100 µL/well, and the plate was incubated at 37°C to allow a chromogenic reaction for 10 min; the chromogenic reaction was stopped, and a value was read. After the purification, when the antibody was diluted to 1 mg/ml, a detected titer reached more than 1:640,000.

### 2.3 Identification of antibody purity and molecular weight

SDS-PAGE was used to identify the molecular weight and purity of the antibody. Gels were prepared, where a separation gel was 12% and a stacking gel was 5%; 20 µL of a sample and 20 µL of a buffer were thoroughly mixed and boiled for 3 min to obtain a sample solution; 20 µL of the sample solution was loaded in each well, and a prestained protein Marker control was set; electrophoresis was conducted at 80 V for 30 min and at 120 V for 2 h; after the electrophoresis was completed, a Coomassie brilliant blue (CBB) solution was added for staining; destaining was conducted by boiling in deionized water 3 times, with 5 min each time; a purified mAb was identified by SDS-PAGE, and resulting bands were clear, without impurity bands. As shown in FIG. 2, there were clear bands at 50 KDa and 25 KDa.

### Example 3: Gene verification of the anti-OXA-23-type carbapenemase antibody

An Ig variable region gene was cloned by RT-PCR. Total RNA was extracted to synthesize single-stranded cDNA. Total RNA was extracted from the hybridoma cell lines HB12 and FA2 by the Trizol method (purchased from Invitrogen), and was reverse-transcribed into a cDNA library with M-MLV reverse transcriptase (purchased from Invitrogen).
Upstream primer for a heavy chain framework region
P1: 5'SAGGTGMAGCTKCASSARTCWGG3', as shown in SEQ ID NO: 21
Downstream primer for a heavy chain variable region
P2: 5'TGGGGSTGTYGTTTTGGCTGMRGAGACRGTGA3', as shown in SEQ ID NO: 22
Upstream primer for a light chain leader peptide
P3: 5'ATGGATTTTCAAGTGCAGATTTTCAG3', as shown in SEQ ID NO: 23
Downstream primer for a light chain variable region
P4: 5'GGATACAGTTGGTGCAGCATCAGCCCGTTT3', as shown in SEQ ID NO: 24
A PCR system (50 µL) was prepared as follows: cDNA: 2 µL; upstream primer (10 µM): 2 µL; downstream primer (10 µM): 2 µL; dNTP mixture: 2 µL; pfu DNA polymerase (5 U/µL): 1 µL; 10 X pfu Buffer II: 5 µL; and ddH₂O: making up to 50 µL.

PCR conditions were as follows: pre-denaturation at 95°C for 5 min; repeating the following cycle 35 times: 95°C for 30 s, 58°C for 30 s, and 72°C for 1 min; and finally, extension at 72°C for 10 min.

VL and VH fragments were separated and recovered by agarose gel electrophoresis. The recovered VL and VH fragments were ligated with a pMD19-T (simple) vector (Takara), and a ligation system was as follows: VL PCR product/VH PCR product: each 70 ng; pMD19-T (simple) vector: 1 µL; Solution I ligation reaction solution: 5 µL; and ddH₂O: making up to 10 µL.

The ligation was conducted at 4°C overnight.

A ligation product was transformed into *E. coli* DH5α competent cells, the competent cells were cultivated overnight at 37°C, single colonies were picked and cultivated at 37°C under shaking for 2 h, and then a resulting bacterial solution was subjected to PCR identification. cDNA of a corresponding antibody was used as a positive control. A reaction system (25 µL) was prepared as follows: bacterial solution: 1 µL; upstream primer (10 µM): 1 µL; downstream primer (10 µM): 1 µL; dNTP Mixture (each 2.5 Mm): 2 µL; Taq DNA polymerase (5 U/µL): 0.5 µL; 10 × Taq Buffer (Mg²⁺ plus): 2.5 µL; and water: making up to 25 µL. Reaction conditions were the same as above.

PCR-positive clones were selected for expansion, and a plasmid was extracted from the positive clones with a plasmid extraction kit (Takara) and sequenced. At least 5 clone samples were sequenced for each chain of each antibody until at least three samples had the same sequencing result. The heavy chain and light chain variable region sequences of the antibodies FA2 and HB12 were successfully cloned, which were consistent with the characteristics of typical antibody variable region sequences according to alignment.

### Example 4: Preparation of an OXA-23-type carbapenemase detection kit

An OXA-23-type carbapenemase test card was prepared by a colloidal gold method, and an immune colloidal gold test strip of a double-antibody sandwich method was prepared as follows:
step 1: a 0.1 M K₂CO₃ solution was added to a colloidal gold solution under stirring, a pH was adjusted, and the anti-OXA-23-type carbapenemase mAb FA2 was added; a resulting mixture was stirred, a 10% bovine serum albumin (BSA) solution and 2% PEG 20000 were added, and a resulting mixture was stirred and centrifuged at a low speed to obtain a supernatant; then the supernatant was centrifuged at a high speed to obtain a precipitate, and the precipitate was resuspended with colloidal gold to form a gold-labeled antibody;
step 2: the gold-labeled antibody was sprayed on a glass fiber membrane, and then the glass fiber membrane was oven-dried to form a gold-labeled pad;
step 3: a 1% thimerosal sodium solution was thoroughly mixed with the anti-OXA-23-type carbapenemase mAb HB12 to form a test line coating solution; then PBS and a 1% thimerosal sodium solution were added to the goat anti-mouse IgG, and a resulting mixture was thoroughly mixed to form a control line coating solution; the control line coating solution and the test line coating solution were streaked on a nitrocellulose (NC) membrane, and the NC membrane was oven-dried to obtain a coated membrane; and
step 4: the coated membrane was attached to a backing card, the gold-labeled pad and absorbent paper were lapped on the coated membrane, and a resulting product was laminated and cut to obtain the OXA-23-type carbapenemase test card of the colloidal gold method.

The examples of the present disclosure are described above in detail, which are merely preferred examples of the present disclosure and cannot be construed as limiting the scope of implementation of the present disclosure.

## Claims

1. An anti-OXA-23-type carbapenemase antibody, wherein the anti-OXA-23-type carbapenemase antibody is: an antibody HB12 comprising a light chain variable region and a heavy chain variable region, wherein complementary-determining regions (CDRs) of the light chain variable region comprise three sequences shown in SEQ ID NO: 1, SEQ ID NO: 2, and SEQ ID NO: 3; and CDRs of the heavy chain variable region comprise three sequences shown in SEQ ID NO: 4, SEQ ID NO: 5, and SEQ ID NO: 6, the light chain variable region has an amino acid sequence shown in SEQ ID NO: 7, and the heavy chain variable region has an amino acid sequence shown in SEQ ID NO: 9;
or,
an antibody FA2 comprising a light chain variable region and a heavy chain variable region, wherein CDRs of the light chain variable region comprise three sequences shown in SEQ ID NO: 11, SEQ ID NO: 12, and SEQ ID NO: 13; and CDRs of the heavy chain variable region comprise three sequences shown in SEQ ID NO: 14, SEQ ID NO: 15, and SEQ ID NO: 16, the light chain variable region has an amino acid sequence shown in SEQ ID NO: 17, and the heavy chain variable region has an amino acid sequence shown in SEQ ID NO: 19.

2. A nucleic acid, comprising a nucleotide sequence encoding the anti-OXA-23-type carbapenemase antibody according to claim 1.

3. The nucleic acid according to claim 2, wherein a nucleotide sequence of the nucleic acid for encoding the light chain variable region of the antibody HB12 is shown in SEQ ID NO: 8, and a nucleotide sequence of the nucleic acid for encoding the heavy chain variable region of the antibody HB12 is shown in SEQ ID NO: 10;
or,
a nucleotide sequence of the nucleic acid for encoding the light chain variable region of the antibody FA2 is shown in SEQ ID NO: 18, and a nucleotide sequence of the nucleic acid for encoding the heavy chain variable region of the antibody FA2 is shown in SEQ ID NO: 20.

4. Use of the anti-OXA-23-type carbapenemase antibody according to claim 1 in the in vitro detection of an OXA-23-type carbapenemase antigen.

5. The use of the anti-OXA-23-type carbapenemase antibody in the detection of an OXA-23-type carbapenemase antigen according to claim 4, wherein the anti-OXA-23-type carbapenemase antibody is used in an *in vitro* diagnostic kit or a microfluidic chip; and the *in vitro* diagnostic kit is a colloidal gold immunoassay kit, a chemiluminescence kit, a radioimmunoassay kit, an enzyme-linked immunosorbent assay (ELISA) kit, or a fluoroimmunoassay kit.

6. The use of the anti-OXA-23-type carbapenemase antibody in the detection of an OXA-23-type carbapenemase antigen according to claim 4,
wherein an immune colloidal gold test strip of a double-antibody sandwich method is prepared, wherein the antibody HB12 is used as a coating antibody and the antibody FA2 is used as a gold-labeled antibody; or
the antibody HB12 is used as a gold-labeled antibody and the antibody FA2 is used as a coating antibody.

## Patentansprüche

1. Carbapenemase-Antikörper vom Anti-OXA-23-Typ, wobei der Carbapenemase-Antikörper vom Anti-OXA-23-Typ ein Antikörper HB12 ist, umfassend eine variable Region der leichten Kette und eine variable Region der schweren Kette, wobei die komplementärbestimmenden Regionen (CDRs) der variablen Region der leichten Kette drei Sequenzen umfassen, die gezeigt ist in SEQ ID NO: 1, SEQ ID NO: 2, und SEQ ID NO: 3; und die CDRs der variablen Region der schweren Kette umfassen drei Sequenzen, die gezeigt ist in SEQ ID NO: 4, SEQ ID NO: 5, und SEQ ID NO: 6, die variable Region der leichten Kette weist eine Aminosäuresequenz auf, die gezeigt ist in SEQ ID NO: 7, und die variable Region der schweren Kette eine Aminosäuresequenz aufweist, die gezeigt ist in SEQ ID NO: 9;
oder
einen Antikörper FA2, der eine variable Region der leichten Kette und eine variable Region der schweren Kette umfasst, wobei die CDRs der variablen Region der leichten Kette drei Sequenzen umfassen, die gezeigt werden in SEQ ID NO: 11, SEQ ID NO: 12, und SEQ ID NO: 13; und die CDRs der variablen Region der schweren Kette umfassen drei Sequenzen, die gezeigt ist in SEQ ID NO: 14, SEQ ID NO: 15, und SEQ ID NO: 16, die variable Region der leichten Kette weist eine Aminosäuresequenz auf, die gezeigt ist in SEQ ID NO: 17, und die variable Region der schweren Kette eine Aminosäuresequenz aufweist, die gezeigt ist in SEQ ID NO: 19.

2. Eine Nukleinsäure, umfassend eine Nukleotidsequenz, die den Anti-OXA-23-Typ-Carbapenemase-Antikörper nach Anspruch 1 kodiert.

3. Nukleinsäure nach Anspruch 2, wobei eine Nukleotidsequenz der Nukleinsäure zum Kodieren der variablen Region der leichten Kette des Antikörpers HB12, die gezeigt ist in SEQ ID NO: 8, und eine Nukleotidsequenz der Nukleinsäure zum Kodieren der variablen Region der schweren Kette des Antikörpers HB12, die gezeigt ist in SEQ ID NO: 10;
oder
eine Nukleotidsequenz der Nukleinsäure zum Kodieren der variablen Region der leichten Kette des Antikörpers FA2, die gezeigt ist in SEQ ID NO: 18, und eine Nukleotidsequenz der Nukleinsäure, die für die variable Region der schweren Kette des Antikörpers FA2 kodiert, die gezeigt ist in SEQ ID NO: 20.

4. Anwendung des Anti-OXA-23-Typ-Carbapenemase-Antikörpers nach Anspruch 1 bei dem In-vitro-Nachweis eines OXA-23-Typ-Carbapenemase-Antigens.

5. Die Anwendung des Anti-OXA-23-Typ-Carbapenemase-Antikörpers zum Nachweis eines OXA-23-Typ-Carbapenemase-Antigens nach Anspruch 4, wobei der Anti-OXA-23-Typ-Carbapenemase-Antikörper in einem In-vitro-Diagnostik-Kit oder einem mikrofluidischen Chip verwendet wird; und es sich bei dem In-vitro-Diagnostik-Kit um ein Kolloidalgold-Immunoassay-Kit, ein Chemilumineszenz-Kit, ein Radioimmunoassay-Kit, ein Enzym-Linked-Immunosorbent-Assay (ELISA)-Kit oder ein Fluorimmunoassay-Kit handelt.

6. Die Anwendung des Anti-OXA-23-Typ-Carbapenemase-Antikörpers zum Nachweis eines OXA-23-Typ-Carbapenemase-Antigens gemäß Anspruch 4, wobei ein immuner kolloidaler Goldteststreifen eines Doppel-Antikörper-Sandwich-Verfahrens vorbereitet wird, wobei der Antikörper HB12 als Beschichtungs-Antikörper und der Antikörper FA2 als goldmarkierter Antikörper verwendet wird; oder
der Antikörper HB12 als goldmarkierter Antikörper und der Antikörper FA2 als Beschichtungs-Antikörper verwendet.

## Revendications

1. Anticorps anti-carbapénémase de type OXA-23, dans lequel l'anticorps anti-carbapénémase de type OXA-23 est : un anticorps HB12 comprenant une région variable de chaîne légère et une région variable de chaîne lourde, dans lequel les régions de détermination complémentaire (CDRs) de la région variable de chaîne légère comprennent trois séquences représentées dans SEQ ID NO : 1, SEQ ID NO : 2, et SEQ ID NO : 3 ; les CDRs de la région variable de chaîne lourde comprend trois séquences représentées dans SEQ ID NO : 4, SEQ ID NO : 5, et SEQ ID NO : 6, la région variable de chaîne légère a une séquence d'acides aminés représentée dans SEQ ID NO : 7, et la région variable de chaîne lourde a une séquence d'acides aminés représentée dans SEQ ID NO : 9 ;
ou,
un anticorps FA2 comprenant une région variable de chaîne légère et une région variable de chaîne lourde, dans lequel les CDRs de la région variable de chaîne légère comprennent trois séquences représentées dans SEQ ID NO : 11, SEQ ID NO : 12, et SEQ ID NO : 13 ; les CDRs de la région variable de chaîne lourde comprend trois séquences représentées dans SEQ ID NO : 14, SEQ ID NO : 15, et SEQ ID NO : 16, la région variable de chaîne légère a une séquence d'acides aminés représentée dans SEQ ID NO : 17, et la région variable de chaîne lourde a une séquence d'acides aminés représentée dans SEQ ID NO : 19.

2. Acide nucléique, comprenant une séquence nucléotidique codant pour l'anticorps anti-carbapénémase de type OXA-23 selon la revendication 1.

3. Acide nucléique selon la revendication 2, dans lequel une séquence nucléotidique de l'acide nucléique pour coder la région variable de chaîne légère de l'anticorps HB12 est représentée dans SEQ ID NO : 8, et une séquence nucléotidique de l'acide nucléique pour coder la région variable de chaîne lourde de l'anticorps HB12 est représentée dans SEQ ID NO : 10 ;
ou,
une séquence nucléotidique de l'acide nucléique pour coder la région variable de chaîne légère de l'anticorps FA2 est représentée dans SEQ ID NO : 18, et une séquence nucléotidique de l'acide nucléique pour coder la région variable de chaîne lourde de l'anticorps FA2 est représentée dans SEQ ID NO : 20.

4. Utilisation de l'anticorps anti-carbapénémase de type OXA-23 selon la revendication 1 dans la détection in vitro d'un antigène de carbapénémase de type OXA-23.

5. Utilisation de l'anticorps anti-carbapénémase de type OXA-23 dans la détection d'un antigène de carbapénémase de type OXA-23 selon la revendication 4, dans lequel l'anticorps anti-carbapénémase de type OXA-23 est utilisé dans un kit de diagnostic in vitro ou une puce microfluidique ; et le kit de diagnostic in vitro est un kit de dosage immunologique d'or colloïdal, un kit de chimioluminescence, un kit de dosage radio-immunologique, un kit de dosage d'immunoabsorption enzymatique (ELISA), ou un kit de dosage immunologique au fluor.

6. Utilisation de l'anticorps anti-carbapénémase de type OXA-23 dans la détection d'un antigène de carbapénémase de type OXA-23 selon la revendication 4, dans lequel une bande de test d'or colloïdal immunitaire d'un procédé sandwich à double anticorps est préparée, dans lequel l'anticorps HB12 est utilisé en tant qu'anticorps de revêtement et l'anticorps FA2 est utilisé en tant qu'anticorps marqué à l'or ; ou
l'anticorps HB12 est utilisé en tant qu'anticorps marqué à l'or et l'anticorps FA2 est utilisé en tant qu'anticorps de revêtement.
